# EUROPEAN PATENT APPLICATION

(11) **EP 4 119 128 A1**
(43) Date of publication of application: **18.01.2023**
(21) Application number: 21185229.8
(22) Date of filing: 13.07.2021
(51) Int. Cl.: A61K 9/14, A61K 9/50, A61K 9/16, A61K 31/5025, A61P 29/00

(54) **PHARMACEUTICAL COMPOSITION FOR THE ORAL ADMINISTRATION OF POORLY SOLUBLE DRUGS COMPRISING AN AMORPHOUS SOLID DISPERSION**

(71) Applicant: Dr. Falk Pharma GmbH, 79108 Freiburg (DE)
(72) Inventor: Stiess, Dr. Michael, 79114 Freiburg (DE); Tewes, Dr. Bernhard, 79279 Vörstetten (DE); Wilhelm, Dr. Rudolph, 76476 Bischweier (DE); Pröls, Dr. Markus, 79100 Freiburg (DE)
(74) Representative: Lederer & Keller Patentanwälte Partnerschaft mbB

(57) **Abstract**

The invention describes the provision of an amorphous solid dispersion of RhuDex^{®} choline salt. The oral, gastro-resistant formulation is provided with a specific release characteristic. Release is realized at pH values from 6 to 7 in the region of the upper intestine. This region is specifically advantageous if pharmaceutical formulations aim to reach the liver as the primary target organ.

## Description

In the course of medical and pharmaceutical research frequently compounds are identified having a very high biological activity connected with the problem of low bioavailability due to the poor solubility of the drug, in particular in aqueous solutions having a physiological pH value. This is frequently coupled with a low permeability of the drug into the target area within the relevant cells.

One compound having promising biological activities is 4-(6-fluoro-3-oxo-1,3-dihydro-pyrazolo[4,3-c]cinnolin-2-yl)-N-(2,2-difluoro-ethyl)-benzamide of the formula (A) 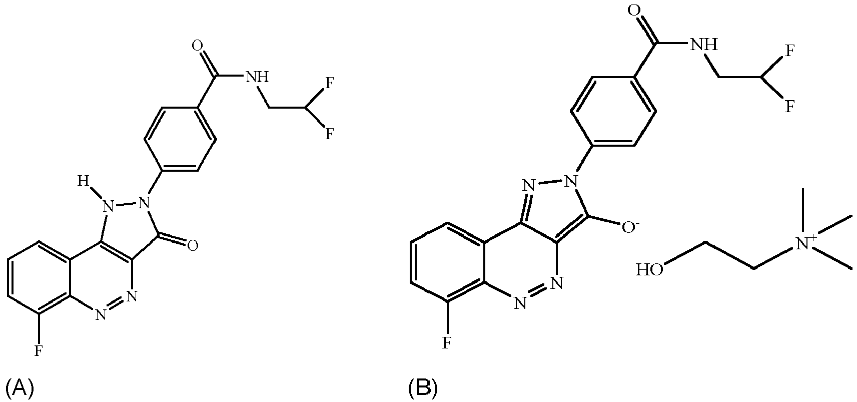 and the choline salt thereof (B). EP 1 991 548 discloses this compound which has been developed by Medigene AG, Munich and is used as injectable aqueous solution under the tradename RhuDex^{®}.

This compound acts as a CD80 antagonist which is capable of inhibiting the interactions between receptor proteins CD28 which are present in the surface of naïve T cells and bind the co-stimulatory ligands B7.1 (CD80) and B7.2 (CD86). Such interaction can be therapeutically used for the treatment of undesired auto-immune reactions, for example in the treatment of rheumatoid arthritis or the treatment of inflammation of the liver. The cell-surface protein CD28 is a co-stimulatory receptor which is present on the surface of all naïve T cells and binds the co-stimulatory ligands now designated as B7.1, former CD80, and B7.2, former CD86. Cell-surface proteins CD28 are expressed mainly on specialized antigen-presenting cells such as dendritic cells. When they become activated, the naïve lymphocyte must engage both antigen and co-stimulatory ligand on the same antigen-presenting cell. CD28 signaling aids antigen-dependent T cell activation mainly by promoting T cell proliferation, cytokine production and cell survival. All these effects are mediated by signaling motifs present in the cytoplasmatic domain of CD28. Although the interaction between CD28 and the co-stimulatory ligands (CD80) do usually have an important and desired effect in the initiation of adaptive immunity by activating T cells, it may in some cases be desirable to block or to lower such immune reactions resulting in inflammation in such diseases wherein unspecific inflammatory reactions occur in the human body. Such undesired inflammatory reactions may be for example rheumatoid arthritis or diseases having auto-immune aspects like Morbus Crohn or inflammatory conditions in hepatology.

Since the parenteral application of such pharmaceutically active drugs is frequently difficult, it is usually preferred to provide an oral delivery system, which must present the drug in a form having sufficient solubility and permeability in the gastrointestinal tract.

The present invention provides a pharmaceutical composition for the oral administration of a drug which is poorly soluble. The composition comprises an amorphous solid dispersion of at least one polymer selected from the group consisting of hypromellose acetate succinate, hypromellose phthalate, methacrylic acid-methylmethacrylate copolymers, povidone, cellulose acetate phthalate, copovidone, polyvinylpyrrolidone-vinyl acetate copolymers and polyethylene glycol, polyvinyl acetate or polyvinyl capalactase based copolymers and an antagonist of the T cell costimulatory protein CD28, such as RhuDex^{®}.

Amorphous solid dispersion (ASD) is a formulation approach aimed to increase the bioavailability of poorly soluble drugs. Besides ensuring physical stability of the molecularly dispersed drug within the polymeric matrix, ASDs can exhibit two decisive features which are relevant for their performance. First, the generation of a supersaturation in a decent amount of time upon dissolution by a suitable dissolution rate and second, the ability to maintain supersaturation for a sufficient duration which can be achieved by precipitation inhibition. These effects must be achieved in the gastrointestinal tract which is characterized by changes of the pH value from acidic in the stomach to nearly neutral in the intestine up to slightly alkaline pH values in the caecum.

In a particularly preferred embodiment of the present invention pharmaceutically acceptable grades of hydroxypropylmethylcellulose acetate succinate (HPMCAS) are used as amorphous solid dispersion with the drug to be applied. Amorphous solid dispersions can be prepared by extrusion methods, in particular hot melt extrusion processes. In such extrusion methods the drug and the polymer are mixed and said mixture is pressed with the help of a suitable extruder through a ring-die having a diameter of 0.1 mm to 2.5 mm whereby the narrower diameters are preferred. The lower limit of the diameter depends, however, on the extruder and the die. Pellets which are extruded can afterwards be spheronized and the pellets obtained therefrom can be dried and sieved to select the desired range of the pellets which may have diameters in the range from 0.1 to 2.5 mm. Furthermore, the pellets can be coated to achieve a certain release profile that allows the targeted delivery of the drug substance to the required site of action or availability in the gastrointestinal tract. The coating of pellets is well-known in the art. It is possible to use coatings which have an influence on the dissolution of the pellets. Either neutral coatings can be used which delay somewhat the dissolution of the pellets or the pellets can be coated with polymers which dissolve depending on the pH value. Some coatings do not dissolve below a pH value of about 6.5 to 6.8 having the consequence that the pellets are not dissolved in the stomach due to the low pH value of about pH 1 to pH 4. It is also possible to coat the pellets with more than one coating, preferably two coatings having different, defined dissolution profiles. Alternatively the ASD can be prepared by special spray-drying methods as described later.

In a particularly preferred embodiment of the present invention the amorphous solid dispersion comprises hypromellose acetate succinate as polymeric carrier and the drug as described herein, shortly designated as RhuDex^{®}.

In another also preferred embodiment of the present invention the pellets containing the drug are prepared by a somewhat different method. Starting with sugar spheres having a size ranging from 0.1 to about 2.0 mm, preferably from 0.15 to 1.0 mm, said parrailles are coated with an amorphous solid dispersion comprising hypromellose acetate succinate and RhuDex^{®}

In a further preferred embodiment the pellets consisting of the sugar pellet coated with the amorphous solid dispersion comprising hypromellose acetate succinate and RhuDex^{®} are coated with a further outer layer which is preferably insoluble at pH values lower than about pH 6.5 to pH 6.8. This has the effect that the pellets are insoluble while remaining in the stomach. After leaving the stomach and entering the small intestine the pH value changes from acidic (pH 1-4) to neutral or near neutral conditions (about pH 6.0-7.2) whereby the outer layer is dissolved rapidly. Then the drug containing layer is dissolved and the drug is set free in a form which is soluble and can be absorbed in the gastrointestinal tract.

Hydroxypropylmethylcellulose acetate succinate (HPMCAS) is available in several different chemical grades which vary in their acetyl and succinoyl group substitution. They have a pH solubility ranging from about 5.5 to slightly above 6.8. In the present invention a pH solubility of about 5.5 to 6.8 is preferred. The molecular weights of the HPMCAS grades range from about 75 kD to about 115 kD.

The term "pellet" as used herein designates comparatively small spherical particles having a diameter ranging from about 0.1 to about 2.2 mm, preferably 0.15 to about 1.0 mm, which contain the pharmaceutically active agent and suitable excipients. The pellets may also be coated with one or more coatings.

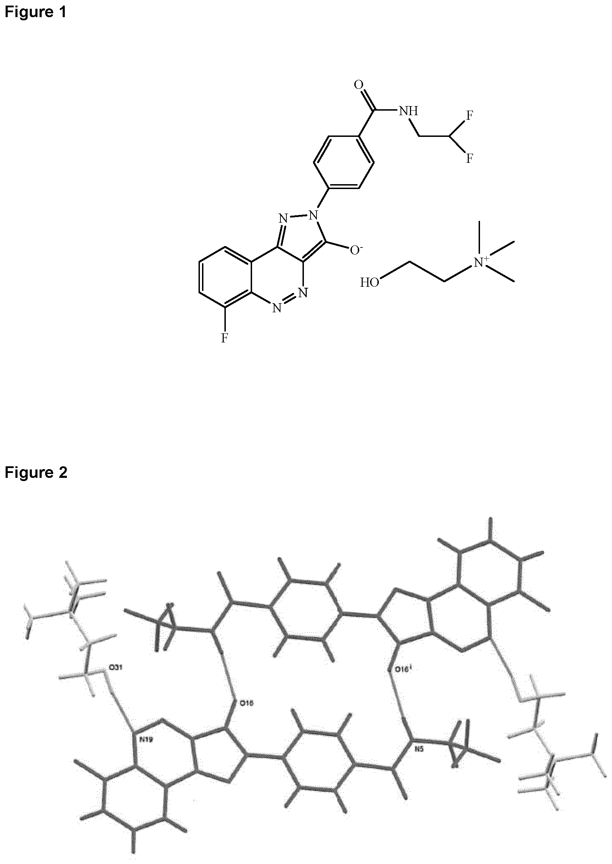

RhuDex^{®} choline salt is a chemically defined, low molecular weight modulator of T-cell activation. It is being developed as an active pharmaceutical ingredient or drug substance for an oral treatment of autoimmune diseases. RhuDex^{®} is the brand name of the free acid that comprises a mono-fluorinated cinnolin and a di-fluorinated benzamide mojety which are connected *via* a pyrazole ring to yield N-(2,2-difluoroethyl)-4-(6-fluoro-3-oxo-1,3-dihydropyrazolo[4,3-c] cinnolin-2-yl)-benzamide. The free acid is the pharmacologically active entity of the compound. It is transferred into the salt at the end of the synthesis by the addition of choline hydroxide (i.e. salification by an acid-base reaction). The molecule is acidic as the negative charge resulting from a deprotonation of the N-H or O-H electrons is resonance stabilised throughout the molecule, which means that the negative charge is delocalised around the aromatic mojety. The resonance structure of RhuDex^{®} choline salt commonly used to define the chemical structure of the drug substance (where the negative charge is located at the oxygen) is given in Figure 2. The compound is achiral with a relative molecular mass of 490 (anhydrous form). The molecular formula of the choline salt is C₂₃H₂₅F₃N₆O₃. After salification and recrystallization the drug substance is micronized by jet milling (D₉₀ ≤ 20 µm).

RhuDex^{®} choline salt is a yellow to red/orange, crystalline solid. The crystal structure has been elucidated by single crystal X-ray diffraction analysis. The results are presented in **Table** 1. The salt consists of a single crystalline phase that crystallizes in the centrosymmetric space group P-1. The crystal form is partially hydrated with about one molecule of water on every three molecules of the RhuDex^{®} choline salt. The water occupies voids in the crystal structure. This partially hydrated form appears to be the thermodynamically most stable crystalline form to exist under ambient conditions. As shown in Figure 2 the ionic interaction between the choline cation and the RhuDex^{®} anion is indicated between oxygen atom 031 and nitrogen atom N19. Oxygen atom 016 is involved in the likely hydrogen bond interaction with the water molecule.

**Table 1: Crystal structure of RhuDex^{®} choline salt by single crystal X-ray diffraction analysis**

| | |
|---|---|
| Empirical molecular formula | C₅H₁₄NO⁺ · C₁₈H₁₁F₃N₃O₂⁻ · 0.33 H₂O¹ |
| Molecular weight | 496.10 |
| T [K] | 296(2) |
| λ [Å] | 0.71073 |
| Crystal system | Triclinic |
| Space group | P-1 |
| Unit cell dimensions | |
| a [Å] | 7.6762(6) |
| b [Å] | 12.1460(9) |
| c [Å] | 12.8554(12) |
| α [Å] | 83.624(5) |
| β [Å] | 77.361(5) |
| γ [Å] | 80.941(5) |
| V [Å³] | 1151.29(17) |
| Z | 2 |
| D_{c} [g/cm³] | 1.431 |
| µ [mm⁻¹] | 0.115 |
| F(000) | 518 |
| Crystal size [mm³] | 0.45 x 0.43 x 0.32 |
| θ range for data collection [°] | 2.9 → 32.5° |
| Reflections collected | 12168 |

| | |
|---|---|
| ¹ In the collected single crystal structure 0.33 molecules of water are present per molecule of RhuDex^{®} choline salt. The gives a water content of 1.2% which correlates well with the water content determined by Karl Fischer titration. | |

RhuDex^{®} choline salt melts at approximately 204°C and the compound is only slightly hygroscopic. The pKa value (free acid) is 5.8.

The aqueous solubility of the crystalline compound within the physiologically relevant pH range from 1 to 7 is extremely low. The saturation solubility of RhuDex^{®} choline salt determined at 37°C after 24 hours by HPLC/UV using the shake-flask method is at around or below 0.001 mg/mL when using standard media or buffers such as 0.1 N hydrochloric acid pH 1.2 and 50 mM phosphate buffer pH 7.0 or biorelevant media such as Fasted-State Simulated Intestinal Fluid (i.e. FaSSIF) pH 6.5 and Fed-State Simulated Intestinal Fluid (FeSSIF) pH 5.0. Acceptable solubility data of the drug substance can only be obtained in water (approximately 10 mg/mL resulting in a final pH value of the solution of 9.9) or in buffer solutions with a pH value greater than 9 (e.g. 50 mM borate buffer, pH 9.5: 1.3 mg/mL). Interestingly, the surfactant (i.e. lecithin) and bile salt (i.e. sodium taurocholate) which are present in the biorelevant media FaSSIF and FeSSIF are insufficient to overcome the strongly pH-dependent solubility of the drug substance.

Considering a minimum solubility of 0.001 mg/mL and a dose of 31.75 mg of RhuDex^{®} choline salt (equivalent to 25 mg of RhuDex^{®} free acid) a volume of more than 30 L would be required to completely dissolve one dose of the drug substance under physiologically relevant conditions.

The biorelevant solubility data of RhuDex^{®} choline acid as well as the qualitative and quantitative composition of the media used in the studies are presented in Table 2. According to the biopharmaceutics classification system (BCS) which is used as an internationally accepted guide for predicting the intestinal adsorption of chemical substances RhuDex^{®} choline salt has to be considered as a low solubility drug belonging to either Class II (low solubility/high permeability) or Class IV (low solubility/low permeability). Drug substances with low aqueous solubility also suffer from poor bioavailability as the drug must be present in the form of a solution to be absorbed at the site of absorption. The BCS criterion for a high solubility drug is only met when the (highest) dose can be dissolved in 250 ml or less of aqueous media over the pH range from 1 to 7.5 at 37°C. Therefore, without improving the solubility of RhuDex^{®} choline salt the required bioavailability to achieve either liver specific targeting or sufficient systemic concentrations for the desired pharmacological response after oral administration would not be feasible.

Under the conditions of the fasted and fed stomach and of the fed upper intestine where the pH value is well below the pKa of RhuDex^{®} choline salt (i.e. pH < 5.8), the ionized and negatively charged molecule is immediately and completely transferred to the neutral free acid that exhibits an extremely low intrinsic solubility leading to the precipitation of the compound. When increasing the pH value above the pKa and beyond the physiological conditions (i.e. pH > 8), the opposite effect occurs as the molecule is transferred into its negatively charged and soluble ion. There is however a small window in-between these two extremes that reflects the physiological conditions of the fasted upper intestine (pH value from 6 to 7) and that offers the opportunity to improve the bioavailability of RhuDex^{®} choline salt by applying solubility enhancing formulation technologies so that a reasonable amount of the drug substance can be dissolved in this area of the gastrointestinal tract in order to significantly improve the compound's bioavailability after oral administration. However, these enabling technologies are neither standard nor easily applicable as they directly affect the physical properties of the drug substance and thereby its thermodynamic stability. Therefore, there is an extremely high risk of failure associated with these technologies concluding that the drug substance cannot be further developed into a suitable, stable, bioavailable and commercially viable formulation.

**Table 2: Saturation solubility of RhuDex^{®} choline salt in biorelevant media (shake-flask method, 24 h at 37°C, quantification by HPLC/UV)**

| **Biorelevant medium** | **Saturation solubility** |
|---|---|
| 0.01 N HCl pH 2 | 0.0001 mg/mL |
| FeSSIF¹, pH 5.0 | 0.0001 mg/mL |
| FaSSIF¹, pH 6.5 | 0.0011 mg/mL |
| Phosphate buffer 50 mM, pH 7.0 | 0.0007 mg/mL |

| | |
|---|---|
| ¹ Fed and fasted-state simulated intestinal fluid composed as follows: | |

The relevant compounds contained within FaSSIF and FeSSIF are as follows:

| | **Amount (mM)** | |
|---|---|---|
| **Component** | FaSSIF | FeSSIF |
| Sodium taurocholate | 3 | 15 |
| Lecithin | 0.75 | 3.75 |
| Sodium hydroxide | 8.7 | 101 |
| Dibasic sodium phosphate (NaH₂PO₄) | 28.65 | --- |
| Acetic acid | --- | 144.05 |
| Sodium chloride | 105.85 | 203.18 |
| pH | 6.5 | 5.0 |

Poor solubility and/or low dissolution rate of potential drug candidates under the physiological conditions of the gastro-intestinal tract limit the compound's bioavailability after oral intake with the consequence that it does not reach the site of action and remains ineffective. Therefore, overcoming the poor aqueous solubility and/or low dissolution rate of a potential drug candidate are major challenges encountered with formulation development of new chemical entities.

In the art of pharmaceutical formulations various highly complex and sophisticated techniques have been developed for the enhancement of either the dissolution rate and/or the solubility of poorly soluble drugs intended to be orally administered. Among these are the following solubilization technologies which can be classified as enabling formulation approaches:
a) The crystalline drug substance can be formulated into a lipid solution containing mixtures of oils, hydrophilic and/or lipophilic surfactants, co-surfactants and co-solvents that support the formation of metastable emulsions or microemulsions once dispersed or diluted in gastric or intestinal fluids (so-called self-emulsifying drug delivery systems or self-microemulsifying drug delivery systems). The solubilized lipid drug substance solution can be filled in softgels that can also be coated.
b) The drug substance can be formulated as lipid based multiparticulates containing the crystalline compound integrated into a matrix of lipophilic compounds, surfactants and co-surfactants that support the formation of metastable emulsions once dispersed or diluted in gastric or intestinal fluids. The multiparticulates can be filled in hard capsules.
c) The crystalline drug substance can be transformed into an amorphous solid dispersion/solution by embedding the compound in a suitable hydrophilic polymer either through spray drying (i.e. solvent evaporation method) or through hot melt extrusion (i.e. melting method). The solid dispersion/solution can be formulated into a variety of different dosage forms. Upon dispersion in gastric or intestinal fluids the thermodynamically activated drug substance and the polymer dissolve and lead to supersaturated and metastable solutions of the drug substance.
d) The crystalline drug substance can be processed with polymers and surfactants into nanosuspensions by suitable milling technologies that reduce the particle size below 500 nm. These nanosuspensions can be further processed, e.g. by layering the nanoparticles on carriers such as sugar spheres. The nanoparticles of the drug substance are still crystalline (i.e. nanocrystals) but have a very high surface area to volume ratio. Thus, the dissolution rate of the drug substance is enhanced and with the support of permeability enhancers that are often added to such formulations the systemic availability of the compound can be increased.

The purpose of all these technologies is to enhance the concentration or fraction of solubilized and bioavailable drug substance in the gastrointestinal fluids. According to the invention amorphous solid dispersions prepared by spray drying or hot melt extrusion technology are preferred as they can create supersaturated solutions where the concentration of the dissolved compound is significantly above its equilibrium saturation solubility. This so-called "spring" effect of the formulated drug substance alone is insufficient to increase the rate and extent of the compound's absorption from the gastrointestinal tract. If not suitably stabilized the drug substance rapidly recrystallizes from the solution until the equilibrium saturation solubility of its more stable crystalline form has been reached again. However, if the supersaturated effect can be maintained sustainably there is a sufficient time window that allows absorption of the poorly soluble drug substance from the intestine during its passage. Therefore, the supersaturated solution of the drug substance must be kinetically stabilized by additional components of the formulation so that its recrystallization can be delayed. This delay is called the "parachute" effect. Only formulations that facilitate both effects, i.e. the spring and the parachute effect, are enabling and there is no general concept available how this spring and parachute effect can be accomplished for a poorly water-soluble drug substance.

Surprisingly, a simple binary mixture comprising the drug substance RhuDex^{®} choline salt and the hydrophilic polymer hypromellose acetate succinate (i.e HPMC-AS) in a weight percent ratio of 1 to 3 (RhuDex^{®} choline salt: HPMC-AS) was found to form extremely stable amorphous solid dispersions after spray drying that could be directly applied on sugar spheres in order to obtain drug substance layered beads, pellets or granules. Furthermore, the same polymer (without drug) can also be layered on the beads/pellets/granules to provide enteric protection. Both manufacturing steps, i.e. manufacture of the spray-dried dispersion and enteric coating could be easily conducted in the fluidized bed coater in a continuous process resulting in gastro-resistant RhuDex^{®} choline salt multiparticulates. Among the available solubilizing technologies only the approach described by the invention was successful to develop Rhudex^{®} choline salt as an enabling, stable and commercially viable formulation. Interestingly, despite the molecule's unfavourable physico-chemical properties the application of a very simplistic but integrated development approach provided a drug product with the required bifunctional performance characteristics. There is no premature release of drug substance in the stomach and once the beads/pellets/granules have reached the near neutral or neutral conditions of the upper small intestine the release of amorphous RhuDex^{®} choline salt is initiated with only a very short delay needed to dissolve the gastro-resistant coating. Then, the second functionality, i.e. the spring and parachute effect, creates supersaturated and kinetically stable drug substance solutions for at least 2 hours and RhuDex^{®} choline salt can be absorbed and removed from the equilibrium.

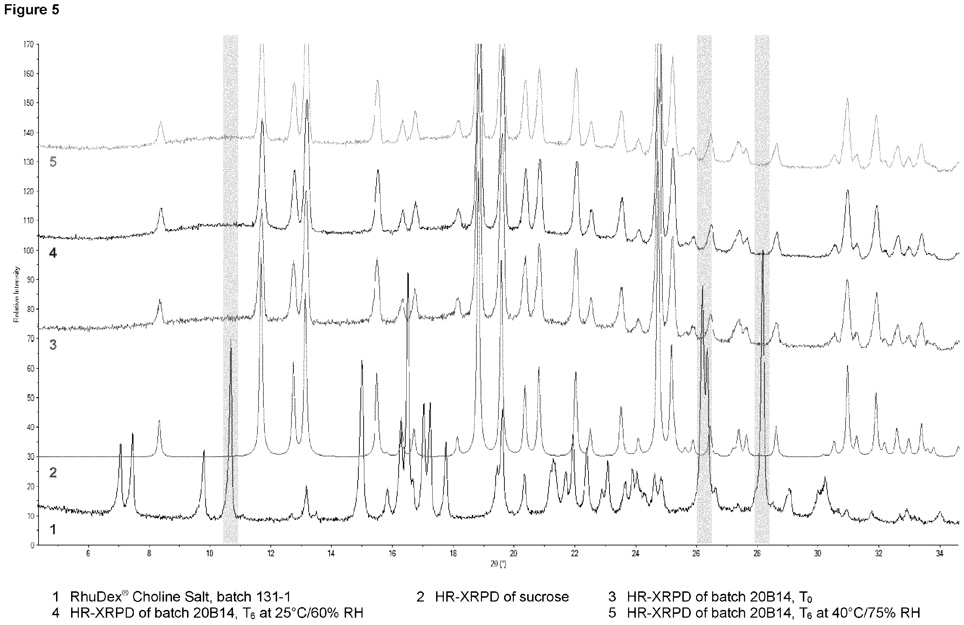

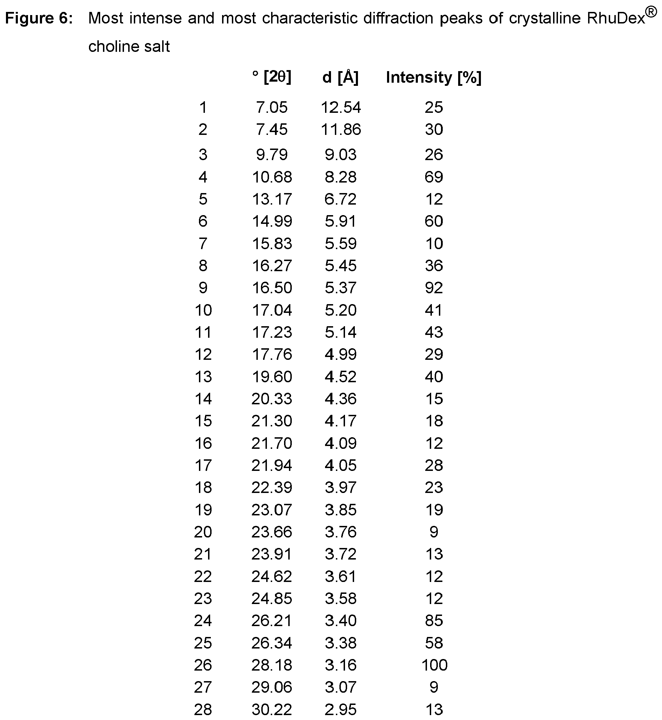

It is especially of note that this surprising effect is maintained even if the samples are stored over a substantial period of time as shown in the Figures 5 and 6.

Amorphous solid dispersions of RhuDex^{®} choline salt can be prepared by spray-drying (i.e. spray-dried dispersions).

This approach assumes that the actual dose of RhuDex^{®} choline salt used is higher than the solubility limited absorbable dose and the absorption is not limited by the compound's dissolution rate but by its solubility in the intestinal lumen. Transforming the crystalline drug substance into an amorphous form and thus breaking the crystal lattice of the molecule is a technique that increases its solubility and leads to supersaturated metastable solutions. Hot-melt extrusion and spray drying are established and preferred technologies to immobilise the drug substance into a rigid water-soluble polymer and to create solid dispersions or solutions. According to the literature this approach can increase the bioavailability by a factor of approximately 20. The main challenges of this approach are to avoid re-crystallisation of the compound, to increase and to maintain supersaturation and to avoid precipitation (i.e. spring and parachute effect). Hot-melt extrusion requires high-shear forces and the application of high local temperature to fuse the drug substance with the polymer. Therefore, thermostability of the compound is an important requisite for this type of technology. Spray-drying technology uses organic solvents to produce a drug substance-polymer solution removing the solvent by a low-temperature evaporation process during spraying. While thermal decomposition of the drug substance is not a critical process parameter, this technology requires special emphasis on the control of the residual solvents in the spray-dried material.

Spray-dried amorphous solid dispersions of RhuDex^{®} choline salt can also be obtained with a number of polymers. These carrier polymers include besides the preferred hypromellose acetate succinate (HPMC-AS), hypromellose phthalate (HPMC-P), methacrylic acid-methyl methacrylate copolymers (Eudragit^{®} L, Eudragit^{®} S), povidone (PVP), cellulose acetate phthalate (CAP), copovidone, polyvinylpyrrolidone-vinyl acetate copolymers (PVPVA) and polyethylene glycol, polyvinyl acetate, polyvinylcaprolactame-based graft copolymer (PVAc-PVCap-PEG, Soluplus^{®}). Solutions of RhuDex^{®} choline salt with one of the polymers in alkaline methanol at different concentrations and varying drug substance-to-polymer ratios can be used for spray drying applications. Solvent evaporation is carried out using conventional spray drying equipments.

Amorphicity can be detected and verified by thermal analysis of the spray-dried powder (e.g. using differential scanning calorimetry) or by dissolution studies. The amorphous solid dispersions of RhuDex^{®} choline salt can be further processed into a variety of suitable oral solid dosage forms. Among these are the manufacture of tablets, especially mini-tablets with a preferred diameter of 1 to 3 mm, using a dry granulation process with subsequent blending, sizing, lubrication and compression steps. Excipients used in these operations are the same as widely established for standard manufacturing processes of tablets. The final powder blends used for compression of the mini-tablets can also be filled into hard capsules of different sizes. Finally coating of the tablets and hard capsules is also feasible to add further functionality, such as gastro-resistance, to the dosage forms.

The most preferred dosage form, however, are RhuDex^{®} choline salt gastro-resistant granules which are obtained by a continuous two-step coating process in a fluidized bed system. Sugar spheres of an appropriate size are used as starter pellets. Preferably the size of the sugar spheres ranges from 850 to 1000 µm. The first layer applied to the pellets comprises the drug substance amorphously embedded in a polymer and the second outer layer which is preferably an enteric coat. Both functionalities can be realized with the same polymer. Preferably, hypromellose acetate succinate is used for both coating steps.

During the coating step the sugar spheres are moved in the fluidized bed of the coater and sprayed with the coating or feeding solution. As the solvent evaporates there is a mass transfer of solid materials from the coating or feeding solution to the sugar spheres thus forming a solid layer on the surface of the pellets. Therefore, the purpose of the solvent is to transport the solids from the coating solution to the surface of the pellets. The spraying or feeding solution of the first and second layer consists of the solute and the solvent in a preferred ratio of 7% w/w for the solute to 93% w/w for the solvent. The solute of the first layer comprises a binary mixture of RhuDex^{®} choline salt and hypromellose acetate succinate in a preferred ratio of about 25% w/w (solute) to about 75% w/w (solvent), while the solute of the second layer comprises hypromellose acetate succinate only. The solvent of both spraying or feeding solutions is a ternary mixture of methanol, purified water and 1 M sodium hydroxide in a ratio of 45 to 65% w/w (methanol) to 25 to 40% w/w (water) to 5 to 15% w/w (1 M sodium hydroxide) resulting in a final pH value of approximately 10.

The conditions in the fluidized bed coater facilitate that the first coating or feeding solution evenly layered on the pellets is spray-dried leading to an amorphous embedment of the drug substance into the carrier polymer. This embedding step was particularly successful when using a weight ratio of about 1 part RhuDex^{®} choline salt to 3 parts hypromellose . The second coating application forms a thin hypromellose film on the pellet surface to achieve gastro-resistant granules.

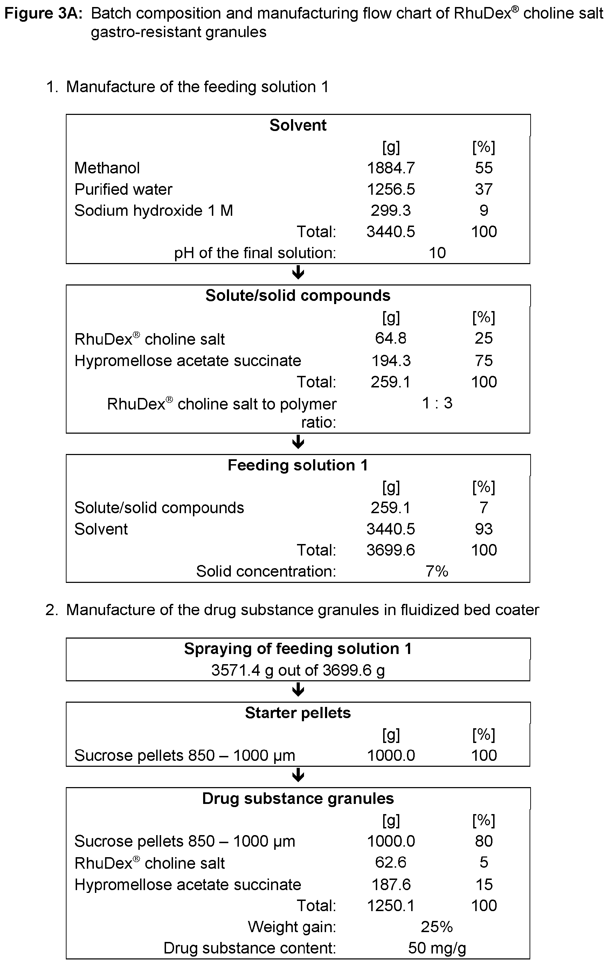

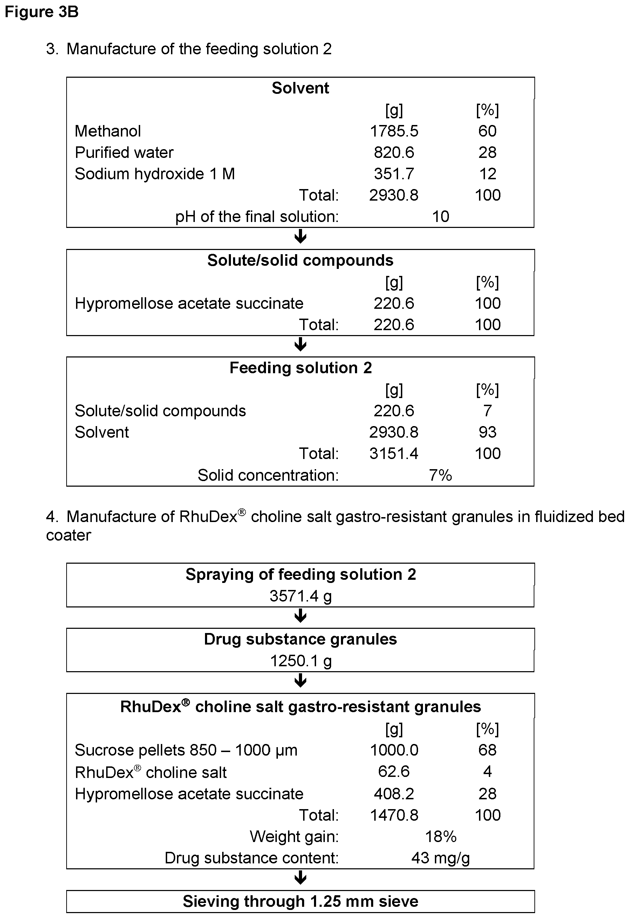

The application of the two layers can be conducted in a fluidized bed coater in a continuous process. Starting with the sugar spheres the first coating leads to drug substance pellets which contain amorphous RhuDex^{®} choline salt and the second coating ensures gastro-resistance. After the second coating step the granules are dried in the coater and then sieved through 1.25 mm. The drug substance concentration of the granules after the first coating step is approximately 50 mg/g and 43 mg/g after the second coating step. The batch composition and manufacturing flow chart of RhuDex^{®} choline salt gastro-resistant granules are presented in Figure 3.

The present invention is further illustrated in the Figures and in the Examples.
Figure 1 shows the chemical structure of the drug substance shortly designated as RhuDex^{®} in the form of a choline salt.
Figure 2 shows the ionic interactions and the hydrogen bond arrangement between symmetry related RhuDex^{®} choline salt molecules. The molecules of the active form and to choline cations are shown whereby hydrogen bonds are also indicated.
Figure 3 A and B shows the batch composition and manufacturing flow chart for the preparation of RhuDex^{®} choline salt gastro-resistant granules in a fluidized bed process.
Figure 4 shows the dissolution profile of an embodiment according to the present invention. After an acidic stage at pH 2.0 whereby no drug is released the buffer is changed. At a pH of 7.0 the dissolved part of the drug is shown with an increase at the spring peak followed by the parachute effect.
Figure 5 shows an overlay of powder X-ray diffraction patterns of stability samples of a batch (18H21) of RhuDex^{®} choline salt gastro-resistant granules, whereby intensity is plotted against the angle of the detector 2θ. The lower curve (1) shows the diffraction pattern of batch 18H21 (RhuDex^{®} choline salt gastro-resistant granules presented in sachets; dose strength: 31.75 mg drug) obtained at time of release. The middle line (2) shows the diffraction pattern of batch 18H21 (RhuDex^{®} choline salt gastro-resistant granules presented in sachets; dose strength 31.75 mg) obtained after a storage period of 24 months at 25°C/60% relative humidity.

The upper diffractogram (3) shows the diffraction pattern obtained from batch 18H21 (RhuDex^{®} choline salt gastro-resistant granules presented in sachets; dose strength: 31.75 mg) after a storage period of 24 months at 2-8°C.

Within the black frame (4) a section of the diffractogram is highlighted where a characteristic diffraction peak of crystalline RhuDex^{®} choline salt should be detectable if present. No disturbing signals could be found. The embodiments of the invention seem to be stable for a storage period of at least 24 months.

Figure 5 shows the overlay of powder X-ray diffraction patterns of crystalline RhuDex^{®} choline salt (with sucrose as comparison) and stability samples of the preferred embodiments of the present invention. Graph (1) shows a diffractogram of crystalline RhuDex^{®} choline salt, batch G02479. Graph (2) shows a powder diffractogram of sucrose which is included for comparative purposes. Graph (3) shows the diffractogram of RhuDex^{®} choline salt gastro-resistant granules, stability batch 16F26 whereby the granules are presented in hard capsules; dose strength: 15.87 mg: To/release data.

Graph (4) of Figure 5 is a diffractogram of RhuDex^{®} choline salt gastro-resistant granules, stability batch 16F26 (granules presented in hard capsules; dose strength: 15.87 mg) whereby 3-month data at 40°C/75% relative humidity (container closure system: HDPE bottle with LDPE cap) are shown.
Graph (5) of Figure 5 shows the diffractogram of RhuDex^{®} choline salt gastro-resistant granules, stability batch 16F26 (granules presented in hard capsules; dose strength: 15.87 mg) whereby 3-month data at 25°C60% relative humidity (container closure system: HDPE bottle with LDPE cap) are shown.

Figure 6 shows the most intense and most characteristic diffraction peaks of crystalline RhuDex^{®} choline salt.

The X-ray powder diffraction patterns show that the pharmaceutical compositions according to the present invention have a surprising stability even when the preparation is stored under severe conditions.

### Examples

The indicated batch formula represents an example of a small scale process. However, depending on the capacity of the fluidized bed coater used the batch size can be easily scaled-up to any reasonable batch size.

The multiparticules can be very flexibly transformed into a variety of different dose strengths of RhuDex^{®} choline salt by either encapsulating the required amount of pellets into a hard gelatine or hydroxypropylmethylcellulose (HPMC) capsule or by filling the required amount of pellets into sachets or stick packs.

**Example 1** presents the qualitative and quantitative composition of different dose strengths of RhuDex^{®} choline salt covering a range from 15.87 mg to 127.5 mg. As the pharmacologically active mojety is the free acid and not the choline salt the indicated dose strengths are equivalent to 12.5 mg to 100 mg RhuDex^{®} free acid.

The example also states the different container closure systems that can be used as primary packaging material. Among these are high-density polyethylene (HDPE) plastic containers with low-density polyethylene (LDPE) caps preferably used for hard capsules or sachets consisting of high barrier laminated aluminium foils that can easily be filled with greater amounts of the gastro-resistant pellets.

To characterize the *in vitro* performance of the gastro-resistant granules that contain the RhuDex^{®} choline salt as an amorphous solid dispersion a predictive biorelevant dissolution method has been developed. The design of the method comprises a two-stage *in vitro* dissolution test that simulates the conditions of the gastrointestinal passage. In a first step the formulation is exposed to simulated gastric fluid where the enteric protection provided by the outer hypromellose succinate acetate layer should ensure that there is no premature drug substance release from the granules. The subsequent step simulates the condition of the upper intestine where the enteric polymer should immediately dissolve and where the drug substance release should start. The gastro-resistance and the release profile are measured by the amount of RhuDex^{®} choline salt dissolved in the acid and buffer medium. The established test system relies on the use of standard media and buffer systems without the addition of any surfactants or bile acids. The volume of the media is also significantly lower than for standard pharmacopoeial dissolution tests. The extent of the spring and parachute effect should be measurable in the simulated intestinal fluid. The parameters of the biorelevant dissolution method are presented below. Summarizing the established conditions for testing the biorelevant dissolution of RhuDex^{®} choline salt gastro-resistant granules, the studies are conducted under non-sink conditions as follows:

| | | |
|---|---|---|
| • Apparatus: | Paddle apparatus (Ph. Eur. apparatus 2) | |
| • Dissolution media: | | |
| - Acid stage, 1 h: | Volume: | 310 ml |
| | Composition: | 35 mM NaCl in demineralised water adjusted to pH 2.0 with 1 M HCI |
| - Buffer stage: 2 h | Volume: | 460 ml |
| | Composition: | 310 ml acid phase + 150 mM phosphate buffer pH 7.3 = 460 ml 50 mM phosphate buffer pH 7.0 |
| • Temperature: | 37°C | |
| • Rotation speed: | 75 rpm | |
| • Testing time points: | | |
| - Acid stage: | 55 min | |
| - Buffer stage: | 5/15/30/60/90/120 min (or cumulative: 65/75/90/120/180 min) | |
| • Number of samples: | N = 2 | |
| • Method of analysis: | HPLC/UV at 320 nm, manually sampling | |

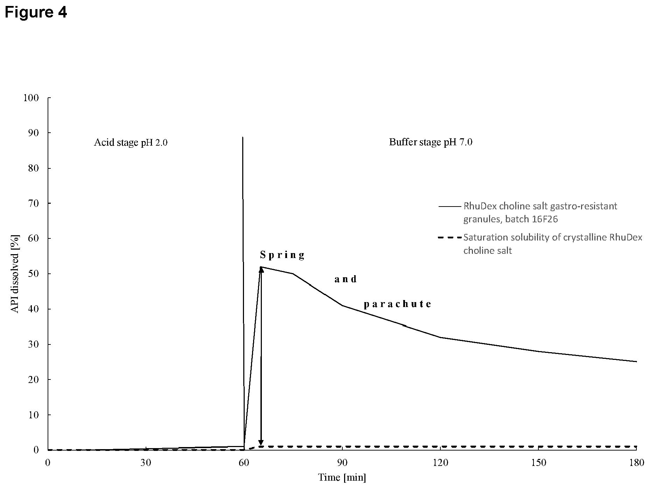

Samples of RhuDex^{®} choline salt 31.75 mg gastro-resistant granules (equivalent to 25 mg RhuDex^{®} free acid; batch 16F26) were tested using the developed biorelevant dissolution method. The dissolution profile is shown in Figure 4. After an exposure of one hour to the acidic medium with a pH value of 2 there is no drug substance release. The granules remain intact and gastro-resistant. When changing to the buffer medium, the enteric layer dissolves and the release of RhuDex^{®} choline salt starts rapidly.

Assuming that the total dose of RhuDex^{®} choline salt (i.e. 31.75 mg) would be completely soluble in the buffer medium of 460 ml a final concentration of 0.07 mg/ml (i.e. 31.75 mg in 460 ml) would result. This theoretical amount represents a complete or 100% release. However, considering the poor solubility of the drug substance at pH 7.0 (approximately 0.001 mg/ml) only 1.4% of the dose would actually be in solution when presented in crystalline form (i.e. 0.001 mg/ml x 460 ml = 0.46 mg). Under these circumstances there would be practically no release of RhuDex^{®} choline salt in the phosphate buffer pH 7.0. When transforming the crystalline drug substance into an amorphous solid dispersion by using the spray drying-technology more than 50% of the dose (i.e. approximately 16.5 mg) are dissolved in the buffer medium leading to highly supersaturated solutions. There is an increase in the solubility by a factor of more than 30-fold which is clearly illustrated by the spring effect as shown in the dissolution profile of the embodiment in Figure 4. According to the invention a solubility of the pharmaceutically active agent can be achieved which is within a range of about 30- to 40-fold of the solubility of the values obtained with other conventional formulations.

After the peak value has been reached the level of supersaturation slightly decreases but the parachute effect can be maintained over 2 hours until the last time point of the dissolution test where still 25% of the dose (i.e. approximately 16 mg) remain in solution indicating that hypromellose acetate succinate functions as a precipitation inhibitor and thus kinetically stabilizing the supersaturated solution of RhuDex^{®} choline salt. Therefore, the biorelevant dissolution data clearly demonstrate that the invented RhuDex^{®} choline salt gastro-resistant granules can produce supersaturated and kinetically stable drug substance solutions.

The biorelevant dissolution profile of the embodiment positively affects the second relevant parameter predicting the compound's oral bioavailability, i.e. its permeability. As shown in apparent permeation studies across monolayers formed by human colon carcinoma cell line Caco-2 the spring and parachute effect observed for formulated RhuDex^{®} choline salt also leads to a higher permeation rate when compared with its crystalline and unformulated counterpart. The permeation assays were performed using an apical buffer at a pH value of 7.0 and a basolateral buffer at a pH value of 7.4. The test concentration was set to 127 µg/ml and should represent a potential clinical dose of 31.75 mg RhuDex^{®} choline salt administrered in a volume of 250 ml (i.e. 31.75 mg/250 ml = 0.127 mg/ml). With an apparent permeation value (Pₐₚₚ) of less than 1 (i.e. 0.12 x 10⁻⁶ cm/s) the permeability of crystalline RhuDex^{®} choline salt across Caco-2 cell monolayers must be classified as poor and a low bioavailability of unformulated drug substance can be estimated. However, the Pₐₚₚ value increased to 2.7 x 10⁻⁶ cm/s when using an amorphous solid dispersion of RhuDex^{®} choline salt in the same experiment indicating a remarkable shift from low to moderate permeability. This effect can be cleary attributed to the embodiment.

### Example 2

RhuDex^{®} choline salt gastro-resistant granules are mono-crystalline. Therefore, powder X-ray diffraction (XRPD) can be used to confirm the amorphous nature of the drug substance in the formulation. The transformation from its crystalline to its amorphous state is associated with the loss of distinctive peaks in the diffractogram so that the remaining peaks are identical with the peaks that belong to sucrose which is the only crystalline compound in the drug product. The following XRPD method (on reflection mode) was used to determine the "amorphicity" of the embodiment:

| | |
|---|---|
| • Sample preparation: | One layer of RhuDex^{®} choline gastro-resistant granules with no additional preparation was placed on the sample holder (Si low background sample holder for small amounts, ∅ 51.5 mm, ∅ 24.5 mm Si crystal). A thin layer of Korasilon paste was placed on the Si crystal to fix the granules for the measurement (preventing the sample from falling off the sample holder). |
| • Measurement: | |
| - Model: | D8 Advance (Bruker) |
| - Cu Kα₁ | |
| - X-ray wavelength: | Cu Kα₁ (Å): 1.54056 |
| | Monochromator: Germanium crystal [1 1 1] |
| - X-ray tube setting: | 40 kV, 40 mA |
| - Detector: | PSD LynxEye |
| - PSD electronic window (°2 Theta): | 3 |
| - Solar slits (°): | 2.5 |
| - Detector slit (mm): | 10.46 |
| - Antiscatering slit (mm): | 6.51 |
| - Scan Mode: | Locked Coupled |
| - Divergence slits: | 0.85 |
| - Capillary size (mm²): | N/A |
| - Scan range (°2 Theta): | 1.5 (2.6; 4.0)-41.4 |
| - Scan step time(s): | 5 |
| - Scan size (°2 Theta): | 0.016 |
| - Number of steps: | 2487 (2441; 2287) |

As demonstrated in Figure 5 the diffraction pattern of a stability batch of the embodiment remains unchanged upon storage. There is a complete match of the diffractograms measured at time of study start and at the end of the accelerated stability study (i.e. 6 months at 40°C/75% relative humidity). The peaks of graph 2 represent the pattern of crystalline sucrose. In the mixture with sucrose the most intense peaks of crystalline RhuDex^{®} choline salt are visible. The diffraction peaks at d = 8.28 ± 0.02 Å (2θ = 10.68 ± 0.05° for cooper radiation) with a relative intensity of 69%, d = 3.40 ± 0.02 Å (2θ = 26.21 ± 0.05° for cooper radiation) with a relative intensity of 85% and d = 3.16 ± 0.02 Å (2θ = 28.18 ± 0.05° for cooper radiation) with a relative intensity of 100% which would be characteristic for crystalline RhuDex^{®} choline salt (see graph 1) are missing in the diffractograms of the stability samples of the embodiment (see graphs 3-5). As the drug substance is amorphous it does not contribute to the diffraction of X-radiation. This is highlighted in Figure 5 by the coloured bars which refer to the most intense diffraction peaks of the crystalline drug substance (see graph 1). The embodiment remains physically stable upon storage and there are no signs of re-crystallisation of the amorphous drug substance to its thermodynamically more stable crystalline form which would be detectable by PXRD.

The table in Figure 6 shows all, i.e. the most intense and the most characteristic, diffraction peaks diffraction peaks of crystalline RhuDex^{®} choline salt.

### Example 3: Qualitative and quantitative composition of different dose strengths of RhuDex^{®} choline salt gastro-resistant granules. The granules were prepared as shown in Fig. 3A and B.

1. One cyclindrical orange hard capsule of size 0 with 15.87 mg RhuDex^{®} choline (equivalent to 12.5 mg RhuDex^{®} free acid) contains 373.42 mg gastro-resistant granules with the following composition:

| **Component** | **Function** | **Quantity per capsule** | |
|---|---|---|---|
| | | [mg] | [%] |
| *Drug substance granules* | | | |
| Sucrose pellets 850 - 1000 µm | Starter pellets | 253.92 | 68.0 |
| RhuDex^{®} choline salt | Drug substance | 15.87 | 4.2 |
| Hypromellose acetate succinate | Hydrophilic polymer | 47.61 | 12.7 |

| *Gastro-resistant granules* | | | |
|---|---|---|---|
| Hypromellose acetate succinate | Enteric protection | 56.61 | 15.0 |
| Sum: | --- | 373.42 | 100.0 |

| *Hard capsule shell* | | | |
|---|---|---|---|
| Gelatin | Structure | 96.36 | --- |
| Titanium dioxide | Opacifier | 0.48 | --- |
| Red iron oxide | Colour pigment | 1.16 | --- |
| Sum hard capsule shell: | --- | 98.00 | --- |

Container closure system: HPDE plastic container (15 ml) with a white LDPE cap (30 mm).
2. One white laminated film sachet with 31.75 mg RhuDex^{®} choline (equivalent to 25 mg RhuDex^{®} free acid) contains 747.08 gastro-resistant granules with the following composition:

| **Component** | **Function** | **Quantity per capsule** | |
|---|---|---|---|
| | | [mg] | [%] |
| *Drug substance granules* | | | |
| Sucrose pellets 850 - 1000 µm | Starter pellets | 508.0 | 68.0 |
| RhuDex^{®} choline salt | Drug substance | 31.75 | 4.2 |
| Hypromellose acetate succinate | Hydrophilic polymer | 95.25 | 12.7 |

| *Gastro-resistant granules* | | | |
|---|---|---|---|
| Hypromellose acetate succinate | Enteric protection | 112.08 | 15.0 |
| Sum: | --- | 747.08 | 100.0 |

Container closure system: One white sachet consists of an amuminium foil internally laminated with a white polyethylene (PE) sealant layer and outside laminated with linear low density polyethylene (LLDPE) and printable polyester (PET).
3. One white laminated film sachet with 63.5 mg RhuDex^{®} choline (equivalent to 50 mg RhuDex^{®} free acid) contains 1494.2 mg gastro-resistant granules with the following composition:

| **Component** | **Function** | **Quantity per capsule** | |
|---|---|---|---|
| | | [mg] | [%] |
| *Drug substance granules* | | | |
| Sucrose pellets 850 - 1000 µm | Starter pellets | 1016.0 | 68.0 |
| RhuDex^{®} choline salt | Drug substance | 63.5 | 4.2 |
| Hypromellose acetate succinate | Hydrophilic polymer | 190.5 | 12.7 |

| *Gastro-resistant granules* | | | |
|---|---|---|---|
| Hypromellose acetate succinate | Enteric protection | 224.2 | 15.0 |
| Sum: | --- | 1494.2 | 100.0 |

Container closure system: One white sachet consists of an amuminium foil internally laminated with a white polyethylene (PE) sealant layer and outside laminated with linear low density polyethylene (LLDPE) and printable polyester (PET).
4. One white laminated film sachet with 127.5 mg RhuDex^{®} choline (equivalent to 100 mg RhuDex^{®} free acid) contains 3000.1 mg gastro-resistant granules with the following composition:

| **Component** | **Function** | **Quantity per capsule** | |
|---|---|---|---|
| | | [mg] | [%] |
| *Drug substance granules* | | | |
| Sucrose pellets 850 - 1000 µm | Starter pellets | 2040.0 | 68.0 |
| RhuDex^{®} choline salt | Drug substance | 127.5 | 4.2 |
| Hypromellose acetate succinate | Hydrophilic polymer | 382.5 | 12.7 |

| *Gastro-resistant granules* | | | |
|---|---|---|---|
| Hypromellose acetate succinate | Enteric protection | 450.1 | 15.0 |
| Sum: | --- | 3000.1 | 100.0 |

Container closure system: One white sachet consists of an aluminium foil internally laminated with a white polyethylene (PE) sealant layer and outside laminated with linear low density polyethylene (LLDPE) and printable polyester (PET).

### Example 4: Alternative process to obtain amorphous solid dispersions of RhuDex^{®} choline salt

Amorphous solid dispersions of RhuDex^{®} choline salt can also be prepared by extrusion methods, in particular hot melt extrusion processes. This example describes the composition and manufacturing process of a RhuDex^{®} choline salt extrudate that is further compressed into gastro-resistant tablets. The drug substance and the polymer copovidone (a copolymer of 1-vinyl-2-pyrrolidone and vinyl acetate in the mass proportion of 3:2) are mixed with other excipients and the said mixture is melted and pressed by the help of a suitable extruder (product temperature below 125°C) through a ring-die. The cooled extrudate strings are cut in approx. 15 cm pieces, milled and sieved to obtain the extrudate with the amorphous RhuDex^{®} choline salt. Using standard unit operation procedures the milled material is processed to dry granules and to the final blend suitable for compression. Finally, the tablets are coated with the enteric polymer hypromellose acetate succinate to obtain gastro-resistant tablets.
1. Qualitative and quantitative composition of RhuDex^{®} choline salt 31.75 mg gastro-resistant tablets

| **Component** | **Function** | **Quantity per tablet** | |
|---|---|---|---|
| | | [mg] | [%] |
| *Drug substance extrudate* | | | |
| RhuDex^{®} choline salt | Drug substance | 31.75 | 13.8 |
| Copovidone | Hydrophilic polymer | 95.25 | 41.3 |
| Macrogol 1500 | Plasticizer | 12.70 | 5.5 |
| Butylhydroxytoluene | Antioxidant | 2.54 | 1.1 |

| *Tablets* | | | |
|---|---|---|---|
| RhuDex^{®} choline salt | Amorphous solid | 142.24 | 61.7 |
| extrudate, millled | dispersion | | |
| Copovidone | Binder | 21.87 | 9.5 |
| Silica colloidal anhydrous | Glidant | 1.39 | 0.6 |
| Crospovidone | Disintegrant | 51.5 | 22.3 |

| *Gastro-resistant tablets* | | | |
|---|---|---|---|
| RhuDex^{®} choline salt tablets | Cores | 217.00 | 94.1 |
| Hypromellose acetate | Gastro-resistant | 7.50 | 3.3 |
| succinate | coating | | |
| Talc | Glidant | 2.00 | 0.9 |
| Titanium dioxide | Pigment | 1.50 | 0.7 |
| Triethyl citrate | Plasticizer | 2.50 | 1.1 |
| Sodium dodecyl sulphate | Surfactant | 0.20 | 0.1 |
| Sum: | --- | 230.70 | 100 |

2. Flow chart of the manufacturing process of RhuDex^{®} choline salt gastro-resistant tablets

### 2.1 Hot melt extrusion and final blend for compression

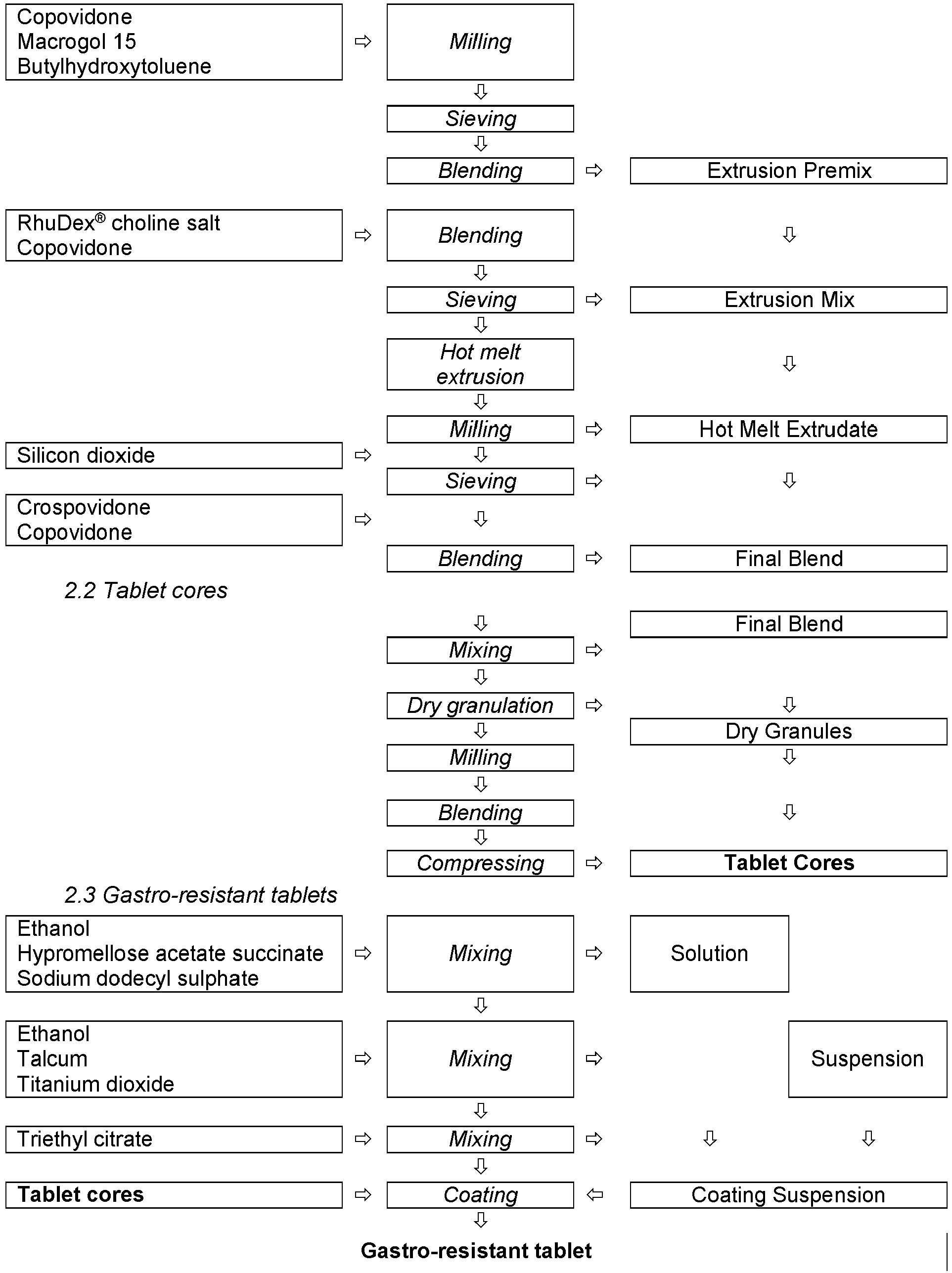

## Claims

1. Pharmaceutical composition for the oral administration of a drug which is poorly soluble in biological fluids which comprises an amorphous solid dispersion of at least one polymer selected from the group consisting of hypromellose acetate succinate, hypromellose phthalate, methacrylic acid-methylmethacrylate copolymers, povidone, cellulose acetate phthalate, copovidone, polyvinylpyrrolidone-vinyl acetate copolymers and polyethylene glycol, polyvinyl acetate or polyvinyl capalactase based copolymers and an antagonist of the T cell costimulatory protein CD28.

2. Pharmaceutical composition according to claim 1 **characterized in that** the polymer is hypromellose acetate succinate.

3. Pharmaceutical composition according to claim 1 or 2 **characterized in that** the antagonist of the T cell costimulatory protein CD28 is 4-(6-fluoro-3-oxo-1,3-dihydro-pyrazolo[4,3-c]cinnolin-2-yl)-N-(2,2-difluoro-ethyl)-benzamide and/or the choline salt thereof: 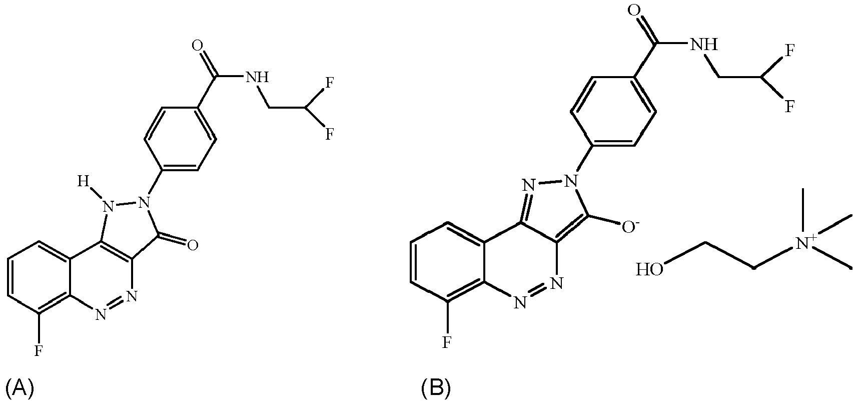 and/or the choline salt (B) thereof.

4. Pharmaceutical composition according to any of claims 1 to 3 **characterized in that** the amorphous solid dispersion consists of hypromellose acetate succinate and RhuDex^{®} choline salt.

5. Pharmaceutical composition according to claim 4 wherein the ratio of the Rhudex^{®} choline salt to the hypromellose acetate succinate is between 1 to 2 up to 1 to 4.

6. Pharmaceutical composition according to claim 5 wherein the ratio of Rhudex^{®} choline salt to the hypromellose acetate succinate is about 1:3.

7. Pharmaceutical composition according to any of claims 1 to 6 **characterized in that** the composition for oral administration is a compressed tablet, extruded granules or spheronized pellets.

8. Pharmaceutical composition according to claims 1 to 6 **characterized in that** an amorphous solid dispersion comprising the drug is layered onto a sugar pellet.

9. Pharmaceutical composition according to claim 8 **characterized in that** the sugar pellet coated with the amorphous solid dispersion has in addition thereto an enteric coating which does not dissolve at a pH below 6.0.

10. Pharmaceutical composition according to claim 9 **characterized in that** the second, outer coating consists of the polymer as used in the amorphous solid dispersion whereby, however, the outer coating does not contain drug.

11. Pharmaceutical composition for the oral administration which is a capsule filled with granules according to claims 7 to 10.

12. Pharmaceutical composition which is a sachet comprising granules according to claims 7 to 10.

13. Pharmaceutical composition according to any of claims 1 to12 for use in the treatment of liver diseases.

14. Pharmaceutical composition according to any of claims 1 to 12 for use in the treatment of rheumatoid arthritis.
